# EUROPEAN PATENT APPLICATION

(11) **EP 2 399 568 A1**
(43) Date of publication of application: **28.12.2011**
(21) Application number: 10425216.8
(22) Date of filing: 25.06.2010
(51) Int. Cl.: A61K 8/19, A61K 8/21, A61K 8/22, A61K 8/24, A61K 8/34, A61Q 11/00

(54) **Teeth whitening composition and method**

(71) Applicant: OCSLabo-OralCareScienceLabo Sagl, 6900 Lugano (CH)
(72) Inventor: Marino, Elio, 20090 Segrate MI (IT)
(74) Representative: Perani, Aurelio

(57) **Abstract**

A combined preparation is described comprising a first sodium hydroxide and xylitol aqueous solution, a second hydrogen peroxide aqueous solution and a third aqueous solution containing sodium hydroxide, a fluoride salt, hydroxyapatite and xylitol. Such preparation can be advantageously used in teeth whitening cosmetic methods without causing the undesired effects generally associated to the use of hydrogen peroxide.

## Description

### Field of the invention

The present invention relates to a teeth whitening combined preparation and a teeth whitening cosmetic method including the use of such preparation.

### State of the art

The use of hydrogen peroxide as an ingredient in compositions for use in teeth whitening methods has been known for a long time.

The effectiveness of such compositions is higher the higher the concentration of ion peroxide, which is the active species. Hence, a first approach to increase the whitening effect may be to increase the concentration of hydrogen peroxide in the composition. However, it must be considered that the concentration of hydrogen peroxide allowed in toothpastes and mouthwashes is generally 0.1%, a concentration considered safe for the consumer, while concentrations of hydrogen peroxide above 6% are considered not safe, unless it is demonstrated that the amount of hydrogen peroxide actually released in the saliva and on the gums does not exceed that released from products containing 6% hydrogen peroxide. This is due to the fact that administration of compositions containing hydrogen peroxide at a concentration of 0.1 to 6% (v/v) may cause some undesired effects, such as re-exacerbation of gingivitis or other periodontal diseases in predisposed subjects, for example in smokers or subjects consuming considerable amounts of alcohol. Furthermore, particular attention must be paid to the use of hydrogen peroxide-based products immediately before or after treatments of tooth restoration in children and adolescents. Finally, accidental ingestion of hydrogen peroxide-containing products may have irritating effects on the oral cavity and gastrointestinal tract.

A second approach to increase the effectiveness of hydrogen peroxide-based whitening compositions is the addition of an alkalinizing agent. However, due to the scarce stability of hydrogen peroxide in a basic environment, it is not possible to prepare alkaline hydrogen peroxide solutions in advance.

A whitening product consisting of two phases has been placed on the market in the United States: a liquid form composition containing 1.5% hydrogen peroxide and a liquid form composition containing 10% sodium bicarbonate; such compositions are mixed at the time of use through a suitable dispenser.

### Description of the invention

It has now been found that it is possible to significantly increase the concentration of hydrogen peroxide in use to obtain an improved whitening effect without causing the above undesired effects.

In a first aspect, the present invention relates to a teeth whitening combined preparation comprising:
1) an aqueous solution, otherwise called "solution 1" or "conditioning mouthwash", containing sodium hydroxide at a concentration of 0.1 to 0.3% (w/w) and xylitol at a concentration of 0.4 to 0.6% (w/w);
2) an aqueous solution containing hydrogen peroxide at a concentration of 15 to 35% (v/v), otherwise called "solution 2" or "whitening solution";
3) an aqueous solution, otherwise called "solution 3" or "remineralizing mouthwash", containing sodium hydroxide at a concentration of 0.1 to 0.3% (w/w), a fluoride salt at a concentration of 0.4 to 0.6% (w/w), hydroxyapatite at a concentration of 0.4 to 0.6% (w/w) and xylitol at a concentration of 0.4 to 0.6 (w/w).

In solutions 1 and 3 the preferred concentration of sodium hydroxide is 0.2%, that of xylitol is 0.5%, that of the fluoride salt and hydroxyapatite is 0.55%.

The concentration of hydrogen peroxide in the second solution is, according to a first preferred embodiment of the invention, 35%; such concentration is particularly well suited when the whitening treatment is carried out under professional control. In a second embodiment of the invention, solution 2 contains 19% of hydrogen peroxide; such solution is particularly well suited as a maintenance treatment or for at-home use.

Each solution may contain additional ingredients suitable for human administration. In particular, solutions 1 and 3 may also contain moisturizing agents, solubilizing agents, flow agents, preservatives, colours and flavours, while solution 2 may contain ethanol, phosphoric acid and parabens as stabilizing substances. As moisturizing agents sorbitol and glycerine can be mentioned; as solubilizing agents castor oil polyethylene glycols, for example castor oil-PEG 40 and vinylpyrrolidone/ammonium acryloyldimethyltaurate copolymer can be mentioned; as a preservative cetyl-benzalkonium chloride can be mentioned. Advantageously, possible colours will be chosen in order to give the solutions different colourings, to facilitate their recognition by the user.

A preferred example of solution 1 has the following composition:

| **Ingredient** | **Amount (percentage in weight)** |
|---|---|
| Purified water | 91.21 |
| Sorbitol | 3.0 |
| Glycerin | 3.0 |
| Brilliant green bio colour 1% w/w LFS (INCI-EU code Aqua-CI 42090 - CI 19140) | 1.0 |
| Hydrogenated castor oil-PEG 40 | 0.5 |
| Xylitol | 0.5 |
| Sodium hydroxide | 0.2 |
| Benzalkonium chloride | 0.2 |
| Teeth and gums flavour T1000840 | 0.1 |
| Mint flavour GH-1069 | 0.1 |
| Cetyl-pyridinium chloride | 0.07 |
| Sodium saccharine | 0.07 |
| Menthol | 0.05 |
| Total | 100.00 |

A preferred example of solution 2 is the solution commercially known with the trademark Peroxal^{®}.

A preferred example of solution 3 has the following composition:

| **Ingredient** | **Amount (percentage in weight)** |
|---|---|
| Purified water | 89.71 |
| Sorbitol | 3.0 |
| Glycerin | 3.0 |
| Vinylpyrrolidone/acryloyldimethyl taurate copolymer | 0.8 |
| Hydrogenated castor oil derivatized with PEG40 | 0.7 |
| Sodium fluoride | 0.55 |
| Hydroxyapatite | 0.55 |
| Xylitol | 0.5 |
| Sodium hydroxide | 0.2 |
| Bio colour FD&C blue 1-Puricolor ABL 9 (INCI code CI 42090) | 0.2 |
| Benzalkonium Chloride | 0.2 |
| Flavour | 0.2 |
| Mint flavour GH-1069 | 0.2 |
| Cetyl-pyridinium chloride | 0.07 |
| Sodium saccharine | 0.07 |
| Menthol | 0.05 |
| Total | 100.00 |

Solutions 1 - 3 may be readily prepared by a skilled technician by means of conventional methods. The combined preparation according to the invention may be marketed in the form of a kit containing the three solutions in different bottles, the second solution being contained in a bottle suitable to be connected with a supplier device allowing to dispense 50 microliters doses; alternatively, it may be already contained in a supplier bottle allowing to dispense such doses. Furthermore, the kit may possibly contain a lip balm or a protective lip cream. In a second aspect, the invention relates to a teeth whitening cosmetic method including the use of the above-described combined preparation. In particular, the method comprises the following steps:
a) rinsing the mouth with 5 - 30 ml, preferably 15 ml, of solution 1 for 20 - 40 seconds, preferably 30 seconds;
b) repeatedly spraying, preferably for 5 - 10 times, 40 - 60 microliters, preferably 50 microliters, of solution 2 on the teeth and spit;
c) rinsing the mouth with water;
d) repeating steps a) - c) for at least other two times, preferably two times;
e) rinsing the mouth with 5 - 30 ml, preferably with 15 ml, of solution 3 for 20 - 40 seconds, preferably 30 seconds.

The treatment defined at steps a) and e) will be carried out for 5 - 7 consecutive days, but it may also be carried out for a lower or higher number of days, in consideration of the pigmentation of the subject's teeth and of the desired degree of whitening.

Additionally, according to a preferred embodiment of the invention, step a) may be advantageously preceded by the application of a protective cream or balm on the lips, in order to protect them from the possible irritating effect of the solutions.

When the treatment is carried out by a professional, a solution 2 containing 35% of hydrogen peroxide will be used, while when the treatment is carried out as a maintenance and/or at-home treatment, a solution 2 containing 19% hydrogen peroxide will be used.

According to a further embodiment of the invention, the method may include a further cycle of maitenance/remineralization to be carried out with solution 3 after carrying out steps a) - e) for a predetermined number of days; such cycle includes the following steps:
f) rinsing the mouth with 5 - 30 ml, preferably 15 ml, of solution 3 for 20 - 40 seconds, preferably 30 seconds;
g) repeatedly spraying, preferably for 10 times, 40 - 60 microliters, preferably 50 microliters, of solution 2 on the teeth and spit;
h) rinsing the mouth with water;
i) rinsing the mouth with 5 - 30 ml, preferably 15 ml, of solution 3 for 20 - 40 seconds, preferably 30 seconds.

The method according to the invention allows to obtain an increase of the whitening effect of at least 50% and up to 2.7 times in comparison with treatments carried out keeping the mouth pH at the normal value of 6.5, without causing the undesired effects commonly associated to whitening with hydrogen peroxide. In particular, neither an increase of dentinal sensitivity nor any phenomenon of enamel erosion were observed.

The improved effect of the combined preparation according to the invention is due to the fact that sodium hydroxide in solution 1 allows to increase the pH of the oral cavity to a value of about 9, thus promoting the dissociation of hydrogen peroxide contained in solution 2 and increasing its effectiveness. In fact, thanks to the administration of solution 1 (conditioning mouthwash), it is possible to administer limited amounts of hydrogen peroxide at high concentrations; for example, performing five sprays of 50 microliters each of hydrogen peroxide 35% solution, the amount of hydrogen peroxide administered amounts to 87.5 microliters, which correspond to 98.87 milligrams. Hence, by performing five sprays of 50 microliters each and by performing three times steps a) - c), an amount of hydrogen peroxide of about 297 milligrams is administered altogether; considering the mean body weight of an individual being 60 kg, the daily administered amount is 0.005 mg/kg, therefore within the established safety limits. Instead, using a 19% hydrogen peroxide solution and repeating three times steps a) - c), the daily administered amount will be below 0.002 mg/kg.

Sodium hydroxide contained in solution 3 allows to complete the whitening effect of solution 2, since it promotes the dissociation of the hydrogen peroxide possibly left in the oral cavity after steps a) - d). Xylitol in solutions 1 and 3 minimizes the risk of enamel erosion and promotes remineralization processes; furthermore, in solution 3, it acts in a synergistic way with the fluoride salt, promoting the deposition of fluorohydroxyapatite in dentinal tubules.

## Claims

1. Teeth whitening combined preparation containing:
a) a first aqueous solution containing sodium hydroxide at a concentration of 0.1 to 0.3% (w/w) and xylitol at a concentration of 0.4 to 0.6% (w/w);
b) a second aqueous solution containing hydrogen peroxide at a concentration of 15 to 35% (v/v);
c) a third aqueous solution containing sodium hydroxide at a concentration of 0.1 to 0.3% (w/w), a fluoride salt at a concentration of 0.4 to 0.6% (w/w), hydroxyapatite at a concentration of 0.4 to 0.6% (w/w) and xylitol a concentration of 0.4 to 0.6% (w/w).

2. Composition according to claim 1 wherein the second solution contains hydrogen peroxide at a concentration of 19% or 35% (v/v).

3. Teeth whitening cosmetic method comprising the following steps:
a) rinsing the mouth for 20 - 40 seconds with 5 - 30 ml of an aqueous solution containing sodium hydroxide at a concentration of 0.1 to 0.3% (w/w) and xylitol at a concentration of 0.4 to 0.6% (w/w);
b) repeatedly spraying 40 - 60 microliters of an aqueous solution containing hydrogen peroxide at a concentration of 15 to 35% (v/v);
c) rinsing the mouth with water;
d) repeating steps a) - c) for at least other two times;
e) rinsing the mouth for 20 - 40 seconds with 5 - 30 ml of an aqueous solution containing sodium hydroxide at a concentration of 0.1 to 0.3% (w/w), a fluoride salt at a concentration of 0.4 to 0.6% (w/w), hydroxyapatite at a concentration of 0.4 to 0.6% (w/w) and xylitol at a concentration of 0.4 to 0.6% (w/w).

4. Method according to claim 3) wherein 15 ml of the first and third solution are used and rinsing is carried out for 30 seconds.

5. Method according to claim 4) wherein the treatment is carried out for 5 to 7 consecutive days.

6. Method according to claim 4) or 5) wherein the hydrogen peroxide concentration is selected from 19% and 35%.

7. Method according to claim 5) or 6) further including a maintenance/remineralization cycle including the following steps:
f) rinsing the mouth with 5 - 30 ml of solution 3 for 20 - 40 seconds;
g) repeatedly spraying 40 - 60 microliters of solution 2 on the teeth and spit;
h) rinsing the mouth with water;
i) rinsing the mouth with 5 - 30 ml of solution 3 for 20 - 40 seconds.
